# EUROPÄISCHE PATENTSCHRIFT

(11) **EP 2 756 791 B1**
(45) Veröffentlichungstag und Bekanntmachung des Hinweises auf die Patenterteilung: **28.07.2021**
(21) Anmeldenummer: 14000164.5
(22) Anmeldetag: 16.01.2014
(51) Int. Cl.: A61B 1/00, A61B 1/12

(54) **Kühleinrichtung für ein Endoskop und/oder ein medizinisches Instrument**
Cooling device for an endoscope and/or medical instrument
Dispositif de refroidissement d'un endoscope et/ou d'un instrument médical

(30) Priorität: 22.01.2013 DE 102013001026
(43) Veröffentlichungstag der Anmeldung: 23.07.2014
(73) Patentinhaber: Karl Storz SE & Co. KG, 78532 Tuttlingen (DE)
(72) Erfinder: Eisele, Hans-Peter, D-78532 Tuttlingen (DE); Schwarz, Peter, D-78532 Tuttlingen-Nendingen (DE)
(74) Vertreter: Weidner Stern Jeschke

(56) Entgegenhaltungen:
- EP-A1- 2 311 366
- DE-A1-102006 002 035
- DE-A1-102010 024 003
- DE-A1-102012 110 143
- JP-A- H09 122 065
- JP-A- 2003 038 437
- JP-A- 2005 040 327
- JP-A- 2006 075 308

## Beschreibung

Die Erfindung betrifft eine endoskopische und/oder medizinische Vorrichtung mit einem Endoskop und/oder einem medizinischen Instrument, wobei das Endoskop bzw. das medizinische Instrument ein Kopfstück aufweist, wobei in dem Endoskop bzw. in dem medizinischen Instrument mindestens ein wärmeerzeugendes Bauteil angeordnet ist und wobei das Kopfstück eine Wärmeaustauschfläche zur Abgabe der von dem mindestens einen wärmeerzeugenden Bauteil erzeugten Wärme nach außerhalb des Endoskops bzw. des medizinischen Instruments aufweist. Die Erfindung betrifft ferner eine Kühleinrichtung für ein derartiges Endoskop bzw. ein derartiges medizinisches Instrument.

Endoskope für medizinische oder technische Anwendungen weisen einen zur Einführung in einen Hohlraum ausgebildeten lang erstreckten Schaft auf, der optische und/oder elektronische Bauteile zur Aufnahme eines Bildes einer Szene in dem Hohlraum und zur Weiterleitung des aufgenommenen Bildes zu einer außerhalb des Hohlraums angeordneten Anzeige oder Betrachtungseinrichtung enthält. Um die aufzunehmende endoskopische Szene ausreichend zu beleuchten, ist es bekannt, in der Nähe des distalen, d.h. benutzerfernen Endes des Schafts eine oder mehrere Lichtquellen, beispielsweise lichtemittierende Dioden (LEDs), anzuordnen. Eine derartige Lichtquelle erzeugt beim Betrieb jedoch Verlustwärme, die aufgrund der Anordnung der Lichtquelle im distalen Endbereich des Schafts zu einer Erwärmung zumindest des distalen Endbereichs des Schafts führt. Diese Erwärmung ist unerwünscht. Insbesondere kann eine Erwärmung der Oberfläche des Schafts über eine Temperatur von 41°C hinaus zu einer Schädigung von Körpergewebe führen, mit dem das Endoskop bei einer medizinischen Anwendung in Kontakt kommt.

Ferner kann ein Endoskop weitere oder andere Wärmequellen aufweisen, etwa elektronische Bildsensoren zur Aufnahme eines endoskopischen Bilds; ebenso kann in einem Lichtanschluss und einem Lichtleiter zur Weiterleitung von Beleuchtungslicht vom proximalen zum distalen Endbereich des Endoskops beim Betrieb des Endoskops Verlustwärme entstehen. Auch medizinische Instrumente, insbesondere bei endoskopischen Operationen eingesetzte medizinische Instrumente, können wärmeerzeugende Bauteile aufweisen, etwa elektrische oder elektronische Bauelemente, Motoren oder andere bewegte Bauteile, die durch ihre Verlustleistung oder durch mechanische Reibung Wärme erzeugen. Die wärmeerzeugenden Bauteile können im distalen Endbereich eines Endoskops oder eines endoskopischen Instruments angeordnet sein und dort in unerwünschter Weise eine Temperaturerhöhung verursachen; die wärmeerzeugenden Bauteile können aber auch in anderen Bereichen eines Endoskops oder eines medizinischen Instruments angeordnet sein und ebenfalls eine unerwünschte Temperaturerhöhung verursachen, die beispielsweise eine Schädigung von Körpergewebe oder von temperaturempfindlichen Bauteilen zur Folge haben oder durch Erwärmung eines Handgriffs die Bedienung des Endoskops bzw. des medizinischen Instruments beeinträchtigen kann.

In der Druckschrift EP 1 738 679 A2 ist eine Kühleinrichtung vorgeschlagen worden, die eine Kühlfluidpumpe, einen ersten Wärmetauscher zur Aufnahme und Abfuhr von Wärmeenergie und einen zweiten Wärmetauscher zur Abgabe der aufgenommenen Wärmeenergie an die Atmosphäre umfasst. Der erste Wärmetauscher befindet sich unmittelbar im Bereich eines wärmeerzeugenden elektrischen Bauteils, etwa einer LED, und ist in einem abkrümmbaren Teil des Endoskopschafts angeordnet. Anstelle des zweiten Wärmetauschers ist es auch möglich, das aufgeheizte Kühlfluid direkt zu entsorgen. Insbesondere bei medizinischen Endoskopen stellt sich hierbei das Problem, dass die mit Kühlfluid beaufschlagten Kanäle der Kühleinrichtung nicht vollständig gereinigt bzw. durch Autoklavieren sterilisiert werden können. Ferner besteht aufgrund der Durchleitung des Kühlfluids durch das Endoskop prinzipiell das Risiko einer Kontamination des Körperinneren eines Patienten, wenn eine Undichtigkeit des Endoskops auftritt.

Gemäß EP 2 394 567 A1 weist ein Endoskop eine in einem distalen Bereich des Schafts angeordnete Lichtquelle auf, die Verlustwärme erzeugt, und eine passive Kühlung, die ein in dem Schaft angeordnetes Wärmerohr in Form einer Heatpipe aufweist, die mit der Lichtquelle thermisch gekoppelt ist, um die Verlustwärme in Richtung nach proximal abzuführen. Das zumindest eine Wärmerohr erstreckt sich bis in das Kopfstück des Endoskops, in dem ein Wärmesenkekörper angeordnet ist, der die Verlustwärme von dem Wärmerohr aufnimmt und direkt oder über ein Gehäuse des Kopfstücks an die Umgebung abgibt.

Aus JP 2003 038437 A ist ein Fiberskop bekannt mit einem Lichtquellenteil, das ein metallisches Teil umfasst, in das eine LED und eine Kondensorlinse eingesetzt sind. Zur Kühlung der LED ist ein Flüssigkeitskreislauf vorgesehen.

Gemäß JP H09 122065 A sind bei einem starren Endoskop Lichtquellen innerhalb eines zylindrischen Handgriffs angeordnet, von wo aus das erzeugte Licht über Lichtleiter durch einen daran anschließenden Schaft zu einer distalen Endfläche weitergeleitet wird. Die von den Lichtquellen erzeugte Wärme wird von einem Peltier-Element zu einer Wärmesenke abgeführt, von wo sie nach außen abgestrahlt wird.

Die Europäische Patentanmeldung EP 2 311 366 A1 beschreibt ein endoskopisches Instrument mit einem LED-Beleuchtungsmodul mit einer am distalen Ende des Instruments angeordneten LED und einer elektrischen Anschlussleitung, die ein Koaxialkabel ist, das zur Abfuhr der von der LED erzeugten Abwärme ausgebildet ist. Die Anschlussleitung weist eine Schnittstelle auf, über die ein am proximalen Ende der Anschlussleitung ggf. noch vorhandener Restanteil der Abwärme abgegeben wird.

In JP 2005 040327 A ist ein elektronisches Endoskop mit einem Festkörper-Bildsensor offenbart, der in der Spitze eines Einführungsteils angeordnet ist und der von einer Treiberschaltung angesteuert wird, die in einem Verbindungsbereich zu einem Videoprozessor aufgenommen ist. Durch einen Lufteinlass wird Druckluft an dem Verbindungsbereich zu einer Luftversorgungsöffnung am distalen Ende des Endoskops geleitet. Vom Lufteinlass zweigt ein Kühlanschluss ab, durch den Druckluft in den Bereich der Treiberschaltung geleitet wird. In DE 10 2006 002 035 A1 ist ein System beschrieben zum Kühlen einer Komponente oder von Komponenten einer Ultraschalltransduceranordnung durch Verwendung eines Dampf/Flüssigkeits-Tiefkühlsystems, welches innerhalb eines Ultraschallbildgebungssystems angeordnet ist, welches Komponenten eines Tiefkühlsystems zur Verbesserung der Wärmeabfuhr integriert. Das Ultraschallsystem weist einen thermisch leitenden Schuh auf, der aus einem festen Material besteht, das geeignet ist zum Kontaktieren oder zum Verbinden mit einem thermisch leitenden Schuh der Transduceranordnung. Dabei liefern die leitenden Schuhe eine thermische Zwischenverbindung, wobei Wärme durch Leitung übertragen wird.

Es ist Aufgabe der vorliegenden Erfindung, eine endoskopische und/oder medizinische Vorrichtung mit einem Endoskop und/oder einem medizinischen Instrument der eingangs genannten Art sowie eine Kühleinrichtung für ein solches Endoskop bzw. ein solches medizinisches Instrument anzugeben, wobei eine effizientere Kühlung eines innerhalb des Endoskops bzw. innerhalb des medizinischen Instruments angeordneten wärmeerzeugenden Bauteils möglich ist, ohne dass hierfür die Durchleitung eines von außen zugeführten Kühlfluids durch im Inneren des Endoskops bzw. des medizinischen Instruments verlaufende Kanäle notwendig ist.

Diese Aufgabe wird durch eine Kühleinrichtung gemäß Anspruch 1 und durch eine endoskopische und/oder medizinische Vorrichtung mit einem Endoskop bzw. einem medizinischen Instrument gemäß Anspruch 2 gelöst.

Vorteilhafte Weiterbildungen der Erfindung ergeben sich aus den Unteransprüchen.

Eine erfindungsgemäße endoskopische und/oder medizinische Vorrichtung umfasst ein Endoskop, insbesondere ein für medizinische Verwendungen geeignetes Endoskop, und/oder ein medizinisches Instrument, insbesondere ein endoskopisches medizinisches Instrument; vorzugsweise ist die endoskopische und/oder medizinische Vorrichtung als endoskopische medizinische Vorrichtung ausgebildet und umfasst ein medizinisches Endoskop oder ein endoskopisches medizinisches Instrument. Das Endoskop bzw. das medizinische Instrument umfasst ein Kopfstück, das eine Wärmeaustauschfläche aufweist, die einen Teil der Oberfläche eines Gehäuses des Kopfstücks bildet. In dem Endoskop bzw. in dem medizinischen Instrument ist mindestens ein wärmeerzeugendes Bauteil angeordnet, beispielsweise eine oder mehrere LEDs oder auch beispielsweise ein elektronischer Bildsensor, etwa ein hochauflösender CMOS-Sensor; auch andere optische, mechanische, elektrische und elektronische Bauteile können Verlustwärme erzeugen, wie etwa ein Lichtanschluss, ein Lichtleiter oder auch ein Motor oder ein anderes bewegtes Bauteil, wobei durch optische bzw. elektrische Verlustleistung oder durch mechanische Reibung Wärme erzeugt wird. Die Wärmeaustauschfläche ist zur Abgabe zumindest eines Teils der von dem mindestens einen wärmeerzeugenden Bauteil erzeugten Wärme nach außerhalb des Endoskops bzw. des medizinischen Instruments ausgebildet.

Innerhalb des Endoskops bzw. des medizinischen Instruments kann ein Wärmeüberträger zur Übertragung mindestens eines Teils der von dem mindestens einen wärmeerzeugenden Bauteil beim Betrieb des Endoskops bzw. des medizinischen Instruments erzeugten Verlustwärme zur Wärmeaustauschfläche des Kopfstücks angeordnet sein. Der Wärmeüberträger ist hierfür mit dem mindestens einen wärmeerzeugenden Bauteil thermisch gekoppelt und ist zur Übertragung der weitergeleiteten Wärme an die Wärmeaustauschfläche mit dieser thermisch gekoppelt. Der Wärmeüberträger kann beispielsweise als aus einem hoch wärmeleitfähigen Material bestehender Draht oder als ein mit einem wärmeübertragenden Fluid gefülltes Wärmerohr, insbesondere als Heatpipe, ausgebildet sein; der Wärmeüberträger kann auch ein Peltier-Element umfassen. Anstelle eines Wärmeüberträgers kann es zur Übertragung der von dem mindestens einen wärmeerzeugenden Bauteil erzeugten Verlustwärme vorgesehen sein, dass das mindestens eine wärmeerzeugende Bauteil direkt mit dem Gehäuse des Kopfstücks bzw. mit der Wärmeaustauschfläche thermisch gekoppelt ist.

Die erfindungsgemäße endoskopische und/oder medizinische Vorrichtung umfasst weiterhin einen Kühlansatz, der mit dem Endoskop bzw. mit dem medizinischen Instrument lösbar verbindbar ist, und der zur Wärmeaufnahme von dem Kopfstück und zur Wärmeabführung mittels eines den Kühlansatz durchströmenden Kühlfluids ausgebildet ist. Der Kühlansatz ist hierfür an das Kopfstück ansetzbar oder auf dieses aufsteckbar, um Wärme von der Wärmeaustauschfläche des Kopfstücks aufzunehmen und diese mit Hilfe des den Kühlansatz durchströmenden Kühlfluids abzuführen. Das Kühlfluid wird über Zu- und Ableitungen, für die der Kühlansatz entsprechende Anschlüsse aufweisen kann, dem Kühlansatz zugeführt bzw. von diesem abgeführt.

Dadurch, dass der Kühlansatz mit dem Kopfstück verbindbar ist zur Aufnahme der zur Wärmeaustauschfläche des Kopfstücks übertragenen Verlustwärme des mindestens einen wärmeerzeugenden Bauteils und zur Abführung der aufgenommenen Wärme durch den Kühlansatz durchströmendes Kühlfluid ausgebildet ist, wird einerseits eine besonders effiziente Kühlung ermöglicht, wodurch eine unzulässige Temperaturerhöhung des Endoskops bzw. des medizinischen Instruments sicher vermieden werden kann und die Abführung auch größerer Mengen an Verlustwärme ermöglicht wird; dadurch können auch aktive Elemente, etwa Peltier-Elemente, für eine besonders wirksame Kühlung und Wärmeübertragung innerhalb des Endoskops bzw. des medizinischen Instruments eingesetzt werden, deren Verlustwärme ebenfalls durch das Kühlfluid abgeführt werden kann. Andererseits ist hierfür keine Durchströmung des Endoskops bzw. des medizinischen Instruments selbst mit dem zugeführten Kühlfluid erforderlich. Eine Reinigung von mit Kühlfluid beaufschlagten Kanälen im Inneren des Endoskops bzw. des medizinischen Instruments ist somit nicht notwendig, ebenso wie ein mit einem Durchströmen des Endoskops bzw. des medizinischen Instruments durch zugeführtes Kühlfluid verbundenes Kontaminationsrisiko weitgehend vermieden werden kann.

Gemäß einer bevorzugten Ausführungsform der erfindungsgemäßen endoskopischen und/oder medizinischen Vorrichtung umfasst diese ein Endoskop und/oder ein medizinisches Instrument mit einen lang erstreckten Schaft, wobei an einem proximalen, d.h. benutzernahen Endbereich des Schafts das Kopfstück angeordnet ist, das die Wärmeaustauschfläche aufweist. Der Schaft ist insbesondere zur Einführung in einen körperinneren Hohlraum geeignet und kann starr, semi-flexibel oder auch flexibel ausgebildet sein. Das mindestens eine wärmeerzeugende Bauteil ist in einem distalen Endbereich des Schafts angeordnet. Innerhalb des Schafts ist ein Wärmeüberträger zur Übertragung der von dem mindestens einen wärmeerzeugenden Bauteil beim Betrieb des Endoskops bzw. des medizinischen Instruments erzeugten Verlustwärme zur Wärmeaustauschfläche des Kopfstücks angeordnet. Der Wärmeüberträger ist hierfür mit dem mindestens einen wärmeerzeugenden Bauteil thermisch gekoppelt und erstreckt sich bis in den proximalen Endbereich des Schafts hinein, insbesondere bis in das Kopfstück, und ist zur Übertragung der weitergeleiteten Wärme an die Wärmeaustauschfläche mit dieser thermisch gekoppelt. Der Wärmeüberträger kann etwa als aus einem hoch wärmeleitfähigen Material bestehender Draht oder als ein mit einem wärmeübertragenden Fluid gefülltes Wärmerohr, insbesondere als Heatpipe, ausgebildet sein; der Wärmeüberträger kann auch ein Peltier-Element umfassen. Dadurch, dass das Kopfstück mit der Wärmeaustauschfläche an einem proximalen Endbereich des Schafts angeordnet ist und dass innerhalb des Schafts ein Wärmeüberträger zur Übertragung der von dem mindestens einen wärmeerzeugenden Bauteil beim Betrieb des Endoskops bzw. des medizinischen Instruments erzeugten Verlustwärme zur Wärmeaustauschfläche des Kopfstücks angeordnet ist, wird eine besonders effiziente Kühlung eines im Schaft, insbesondere im distalen Endbereich des Schafts, angeordneten wärmeerzeugenden Bauteils ermöglicht. Hierdurch kann eine unzulässige Temperaturerhöhung des Schafts, insbesondere des distalen Endbereichs des Schafts, des Endoskops bzw. des medizinischen Instruments sicher vermieden werden.

Vorzugsweise ist das Endoskop bzw. das medizinische Instrument hermetisch dicht ausgebildet und kann somit insbesondere autoklavierbar ausgebildet sein. Hierdurch wird eine besonders sichere Sterilisation ermöglicht. In besonders vorteilhafter Weise ist das Endoskop bzw. das medizinische Instrument als wiederverwendbares, hermetisch dichtes Endoskop bzw. Instrument ausgebildet.

Erfindungsgemäß ist der Kühlansatz als Handgriff ausgebildet. Hierfür kann eine äußere Oberfläche des Kühlansatzes in einer ergonomischen Form, beispielsweise mit Griffmulden, und aus einem für die Verwendung als Handgriff optimalen Material ausgebildet sein. Der Kühlansatz kann ferner Bedienelemente umfassen, etwa zur Ansteuerung einer im Endoskop angeordneten Kamera oder zur Abwinkelung einer Optik bzw. eines distalen Bereichs des Endoskops bzw. des medizinischen Instruments. Hierdurch wird die Bedienung des Endoskops bzw. des medizinischen Instruments für einen Benutzer erleichtert. Dadurch, dass eine Wärmeabführung durch das den Kühlansatz durchströmende Kühlfluid erfolgt, wird es ermöglicht, die Oberfläche des Kühlansatzes unabhängig von einer Temperatur der Wärmeaustauschfläche stets auf einer für die Benutzung als Handgriff günstigen Temperatur zu halten, so dass die äußere Oberfläche des Kühlansatzes zur Handhabung des Endoskops bzw. des medizinischen Instruments von einem Benutzer ergriffen oder umgriffen werden kann.

Gemäß einer bevorzugten Ausführungsform der Erfindung ist der Kühlansatz als Einwegteil ausgebildet. Hierdurch entfällt eine aufwändige Reinigung und Sterilisation des Kühlansatzes nach einer Benutzung bei einer medizinischen Anwendung, die aufgrund der Durchströmung mit Kühlfluid notwendig sein kann. Insbesondere ist es möglich, ein wiederverwendbares, vorzugsweise hermetisch dichtes Endoskop bzw. medizinisches Instrument mit einem zur einmaligen Benutzung bestimmten Kühlansatz, der gleichzeitig als Handgriff ausgebildet sein kann, zu verwenden. Da das Endoskop bzw. das medizinische Instrument aufgrund der darin enthaltenen optischen und elektronischen Bauteile sowie der bei einem in einen körperinneren Hohlraum des Menschen einzuführenden Schaft bestehenden Sicherheitsanforderungen den wesentlichen Anteil der Herstellkosten der endoskopischen und/oder medizinischen Vorrichtung ausmacht, wird hierdurch eine einfache und kostengünstige Benutzung ermöglicht.

Alternativ kann der Kühlansatz für eine mehrfache Verwendung ausgelegt sein, wobei aus Sicherheitsgründen beispielsweise eine zweite Wandung vorgesehen sein kann. Hierbei kann der Kühlansatz in vorteilhafter Weise derart ausgelegt sein, dass eine einfache Reinigung der vom Kühlfluid durchströmten Kanäle möglich ist. Insbesondere sind sowohl das Endoskop als auch der Kühlansatz wiederverwendbar ausgebildet. Hierdurch wird eine kostengünstige mehrfache Benutzung ermöglicht. Ferner ist es insbesondere bei einem wiederverwendbaren Kühlansatz zur Erhöhung der Sicherheit bevorzugt, dass durch ein Sensorsystem eine Leckage der durch den Kühlansatz verlaufenden Kühlleitungen automatisch feststellbar ist und dem Benutzer angezeigt werden kann.

Erfindungsgemäß ist der Kühlansatz zur Durchströmung mit einem in einem geschlossenen Kühlkreislauf geführten Kühlfluid ausgebildet. In einem derartigen geschlossenen Kühlkreislauf ist vorzugsweise eine Pumpe angeordnet, die zur Abführung von Wärme aus dem Kühlansatz das Kühlfluid durch einen im Kühlansatz enthaltenen ersten Wärmetauscher fördert. Der geschlossene Kühlkreislauf kann einen zweiten Wärmetauscher zur Abgabe der vom Kühlfluid abgeführten Wärme an die Umgebungsluft umfassen. Der Kühlansatz weist vorzugsweise Anschlüsse zum Anschluss an Verbindungsleitungen auf, durch die der Kühlkreislauf über den Kühlansatz geschlossen wird. Die Leitungslänge und der Leitungsquerschnitt der durch den Kühlansatz verlaufenden Kühlfluidleitungen sowie die Förderleistung der Pumpe können derart aneinander angepasst sein, dass stets eine zur Wärmeabführung ausreichende Durchströmung mit Kühlfluid gewährleistet ist. Ferner kann eine Steuerungseinrichtung zur Ansteuerung der Pumpe zur Erzeugung eines zur Abführung der aufgenommenen Verlustwärme geeigneten Kühlfluidstroms vorgesehen sein; zur Regelung des Kühlfluidstroms kann innerhalb des Kühlkreislaufs, vorzugsweise im Bereich des Kühlansatzes, ein Temperatursensor angeordnet sein. Hierdurch kann unter einer Vielzahl von Betriebsbedingungen eine stets optimale Wärmeabführung gewährleistet werden.

Gemäß einer bevorzugten Ausführungsform der Erfindung ist der Kühlansatz zur Durchströmung mit einem Kühlfluid unter Schwerkraftwirkung ausbildet. Die Durchströmung mit Kühlfluid kann auch hierbei in einem geschlossenen Kreislauf erfolgen, der durch Konvektion angetrieben wird, wobei die Kühlfluidführung durch den ersten Wärmetauscher entsprechend ausgelegt ist. Die Leitungslänge und der wirksame Strömungsquerschnitt der durch den Kühlansatz verlaufenden Kühlfluidleitungen können hierbei in besonders vorteilhafter Weise aber auch derart gewählt sein, dass bei Entnahme des Kühlfluids aus einem Sterilwasserbeutel unter der Wirkung der dabei mit üblichen Mitteln in einem Operationssaal realisierbaren Potentialdifferenz eine stets ausreichende Wärmeabführung gewährleistet ist; das Kühlfluid wird hierbei nicht in einem geschlossenen Kreislauf geführt. Da die während eines chirurgischen Eingriffs benötigte Kühlwassermenge zur Abführung der dabei entstehenden Verlustwärme aufgrund der hohen Wärmekapazität von Wasser sehr gering ist, reicht eine Vorratsmenge eines üblichen Sterilwasserbeutels von beispielsweise 2 1 in der Regel auch für lange Operationen aus. Am Auslauf aus dem Kühlansatz kann ein Durchflussbegrenzer angeordnet sein, der auf die jeweils gewünschte Durchflussmenge eingestellt werden kann. Hierdurch wird nicht nur ein sehr einfaches und störungssicher arbeitendes System geschaffen, sondern die Verwendung eines sterilen Kühlfluids ermöglicht den zusätzlichen Vorteil, dass selbst bei einer Undichtigkeit des Kühlansatzes oder der Zu- bzw. Ableitungen keine Kontamination des Patienten eintreten kann.

Gemäß einer bevorzugten Ausführungsform der Erfindung weist der Kühlansatz eine Wärmeaustauschfläche auf, die mit der Wärmeaustauschfläche des Kopfstücks zumindest teilweise in thermischen Kontakt gebracht werden kann und die zur Wärmeabführung mit einem von dem Kühlfluid durchströmbaren Wärmetauscher thermisch gekoppelt ist. Insbesondere kann die zweite Wärmeaustauschfläche derart passend zur ersten Wärmeaustauschfläche ausgebildet sein, dass beide Wärmeaustauschflächen durch das Verbinden des Kühlansatzes mit dem Endoskop bzw. mit dem medizinischen Instrument in direkte Anlage miteinander oder auch in eine zueinander dicht benachbarte Anordnung gebracht werden können. Im letzteren Fall kann der zwischen den Wärmeaustauschflächen des Kopfstücks und des Kühlansatzes verbleibende Spalt mit einem wärmeleitfähigen Material ausgefüllt sein. Dadurch, dass die beiden Wärmeaustauschflächen in direkte Anlage miteinander oder in zueinander eng benachbarte Anordnung gebracht werden können, wird auf einfache Weise ein besonders intensiver Wärmeübergang ermöglicht.

In vorteilhafter Weise ist die Wärmeaustauschfläche des Kopfstücks als eine bezogen auf eine Längsachse des Endoskops bzw. des medizinischen Instruments radial äußere Oberfläche des Kopfstücks ausgebildet. Der Kühlansatz kann in diesem Fall insbesondere eine Wärmeaustauschfläche aufweisen, die als hiermit korrespondierende innere Oberfläche einer Hohlstruktur des Kühlansatzes ausgebildet ist. In besonders vorteilhafter Weise ist die Wärmeaustauschfläche des Kopfstücks als Zylindermantel ausgebildet, dessen Achse beispielsweise parallel zu einer Längsachse eines Schafts des Endoskops bzw. des medizinischen Instruments gerichtet ist, und die Wärmeaustauschfläche des Kühlansatzes bildet die Innenfläche einer zylindrischen Hülse. Der Innenradius der zylindrischen Hülse ist dabei gleich oder geringfügig größer als der Außenradius des zylindrischen, als Wärmeaustauschfläche ausgebildeten Bereichs des Kopfstücks. Der Wärmetauscher des Kühlansatzes umgibt die zylindrische Wärmeaustauschfläche des Kühlansatzes nach außen; der Wärmetauscher kann wiederum von einem als Griffschale eines Handgriffs ausgebildeten Gehäuse des Kühlansatzes umgeben sein. Hierdurch wird eine einfache und stabile Anordnung geschaffen, die eine effiziente Wärmeabführung ermöglicht.

Gemäß einer weiteren bevorzugten Ausführungsform bildet die Wärmeaustauschfläche des Kopfstücks mindestens einen Teil einer proximalen Stirnseite des Kopfstücks. Vorzugsweise weist der Kühlansatz eine Wärmeaustauschfläche auf, die mit der proximalen Stirnseite des Kopfstücks in Anlage oder in nahe benachbarte Anordnung bringbar ist. Insbesondere kann die proximale Stirnseite des Kopfstücks mit der Wärmeaustauschfläche des Kopfstücks senkrecht zu einer Längsachse des Schafts angeordnet sein, und die Wärmeaustauschfläche des Kühlansatzes eine ebenfalls senkrecht zur Achse des Schafts stehende, an der Stirnseite des Kopfstücks anliegende oder dieser nahe benachbarte Fläche bilden, wenn der Kühlansatz mit dem Kopfstück verbunden ist. Auch hierdurch ist auf besonders einfache Weise die Erzielung eines wirksamen Wärmeübergangs vom Kopfstück zum Kühlansatz möglich.

Vorzugsweise ist in einem Spalt, der nach dem Verbinden des Kühlansatzes mit dem Kopfstück zwischen der Wärmeaustauschfläche des Kopfstücks und der des Kühlansatzes verbleibt, ein wärmeleitfähiges Material angeordnet, insbesondere ein dauerelastisches Wärmeleitmaterial, etwa mit wärmeleitenden Partikeln gefüllte Kunststoffmatten. Das wärmeleitfähige Material ist in vorteilhafter Weise derart gewählt und bemessen, dass das Material bei dem Betrieb der endoskopischen und/oder medizinischen Vorrichtung aufgrund der übertragenen Wärme und der dadurch veranlassten Temperaturerhöhung der Wärmeaustauschflächen eine Volumenvergrößerung erfährt; das Material kann beispielsweise ein Phase-Change-Material sein. Insbesondere kann das wärmeleitfähige Material auf einer der beiden Wärmeaustauschflächen aufgebracht sein, wobei zwischen der Oberfläche des wärmeleitfähigen Materials und der anderen Wärmeaustauschfläche beim Verbinden des Kühlansatzes mit dem Kopfstück ein Restspalt verbleibt, der das Verbinden des Kühlansatzes und des Kopfstücks erleichtert. In besonders vorteilhafter Weise ist das Material derart gewählt und der Restspalt derart bemessen, dass das Material durch die Temperaturerhöhung beim Betrieb der Vorrichtung eine solche Volumenvergrößerung erfährt, dass der Restspalt geschlossen wird. In weiter vorteilhafter Weise kann es vorgesehen sein, dass die durch die übertragene Wärme verursachte Volumenvergrößerung nicht nur ausreicht, den Restspalt zu schließen, sondern auch zu einem Kraftschluss zwischen dem Kopfstück und dem Kühlansatz über die Wärmeaustauschflächen und das dazwischen angeordnete wärmeleitfähige Material führt. Hierdurch wird eine besonders einfache und sichere Bedienung bei einer besonders wirksamen Wärmeübertragung ermöglicht.

Gemäß einer weiteren bevorzugten Ausführungsform ist auf einer Oberfläche des Kühlansatzes, die nach dem Verbinden des Kühlansatzes mit dem Kopfstück der Wärmeaustauschfläche des Kopfstücks gegenüber liegt, mindestens ein mit dem Kühlfluid befüllbares volumenveränderliches Element, etwa ein Ballon, angeordnet. Zwischen der Oberfläche des Kühlansatzes und der Wärmeaustauschfläche verbleibt dabei ein Spalt, der durch das volumenveränderliche Element in einem nicht mit Kühlfluid befüllten Zustand nur teilweise ausgefüllt wird, so dass ein Verbinden des Kühlansatzes mit dem Kopfstück, etwa ein Aufschieben einer Hülse des Kühlansatzes auf eine zylindrisch geformte Wärmeaustauschfläche des Kopfstücks, nicht behindert wird. Beim Betrieb der Vorrichtung wird das mindestens eine volumenveränderliche Element aufgrund des von einer Kühlfluidpumpe oder durch Schwerkraftwirkung erzeugten Drucks mit Kühlfluid befüllt und dadurch fest an die Wärmeaustauschfläche des Kopfstücks angedrückt. In diesem Fall kann es ausreichend sein, dass lediglich das wenigstens eine mit dem Kühlfluid befüllbare volumenveränderliche Element vom Kühlfluid durchströmt wird, ohne dass ein zusätzlicher Wärmeaustauscher im Kühlansatz vorhanden ist. Der Wärmeübergang vom Kopfstück zum Kühlfluid erfolgt hierbei durch die Wand des mindestens einen volumenveränderlichen Elements, beispielsweise durch die dünne Wand eines Ballons. Auch hierdurch ist auf einfache Weise ein besonders wirksamer Wärmeübergang erzielbar.

In einer weiteren bevorzugten Ausführungsform der Erfindung ist der Kühlansatz derart ausgebildet, dass durch Verbinden des Kühlansatzes mit dem Kopfstück ein fluiddicht abgeschlossener, von dem Kühlfluid durchströmbarer Raum zwischen der Wärmeaustauschfläche des Kopfstücks und einer Oberfläche des Kühlansatzes geschaffen wird. Hierfür kann ein Spalt zwischen der Wärmeaustauschfläche des Kopfstücks und der Oberfläche des Kühlansatzes vorgesehen sein, der von einer oder mehreren Dichtungen begrenzt wird. Auf diese Weise kann ein direkter und somit besonders effizienter Wärmeübergang von der Wärmeaustauschfläche des Kopfstücks zum Kühlfluid erreicht werden. In diesem Fall ist die Verwendung eines sterilen Kühlfluids besonders vorteilhaft, um eine Kontamination des Patienten auch bei einer etwaigen Undichtigkeit der einen oder mehreren Dichtungen zu vermeiden.

In besonders vorteilhafter Weise ist der Kühlansatz mit dem Kopfstück verriegelbar verbindbar. Hierfür kann beispielsweise eine nach Art eines Bajonetts oder einer Schraubverbindung ausgebildete Verriegelung oder eine Rastverbindung vorgesehen sein. Hierdurch wird nicht nur ein sicheres Halten des Kühlansatzes am Kopfstück und damit beispielsweise eine sichere Handhabung des Endoskops bzw. des medizinischen Instruments mit Hilfe eines als Handgriff ausgebildeten Kühlansatzes ermöglicht, sondern es kann der Kühlansatz auch mit einer Anpresskraft an dem Kopfstück fixierbar sein. Hierdurch wird insbesondere in dem Fall, dass die Wärmeaustauschfläche an einer proximalen Stirnseite des Kopfstücks angeordnet ist, ein besonders wirksamer Wärmeübergang ermöglicht.

Gemäß einer bevorzugten Ausführungsform der Erfindung weisen das Kopfstück und der Kühlansatz miteinander zusammenwirkende elektrische Steckverbinderteile auf, die derart ausgebildet sind, dass beim Verbinden des Kühlansatzes mit dem Kopfstück eine elektrische Verbindung zwischen dem Kopfstück und dem Kühlansatz hergestellt wird. Wenn das Verbinden durch eine in axialer Richtung des Schafts gerichtete Bewegung erfolgt, beispielsweise in dem erfindungsgemäßen
Fall, dass die Wärmeaustauschfläche des Kopfstücks einen axial gerichteten Zylinder bildet und der Kühlansatz als auf den Zylinder aufschiebbare Hülse ausgebildet ist, sind die Steckverbinderteile zum Zusammenstecken in axialer Richtung ausgebildet. Vorzugsweise weist der Kühlansatz elektrische Anschlussleitungen oder Anschlüsse zum elektrischen Verbinden mit einer Versorgungseinrichtung der endoskopischen und/oder medizinischen Vorrichtung auf. Hierdurch wird durch das Verbinden des Kühlansatzes mit dem Endoskop bzw. mit dem medizinischen Instrument im gleichen Arbeitsschritt die elektrische Verbindung zur Versorgung des Endoskops bzw. des medizinischen Instruments sowie ggf. zum Weiterleiten eines aufgenommenen endoskopischen Bilds zu einer Anzeigeeinrichtung hergestellt.

Eine erfindungsgemäße Kühleinrichtung für eine gattungsgemäße endoskopische und/oder medizinische Vorrichtung ist als Kühlansatz ausgebildet, der zur Wärmeaufnahme von der Wärmeaustauschfläche des Kopfstücks mit dem Endoskop bzw. mit dem medizinischen Instrument verbindbar ist und der zur Wärmeabführung durch ein den Kühlansatz durchströmendes Kühlfluid ausgebildet ist. Insbesondere weist der Kühlansatz die oben beschriebenen Merkmale des Kühlansatzes auf. Hierdurch wird eine Kühleinrichtung geschaffen, die einfach handhabbar ist und die eine effiziente Abführung der in einem Endoskop bzw. in einem medizinischen Instrument der genannten Art erzeugten Verlustwärme ermöglicht.

Die Erfindung betrifft ferner ein medizinisches Instrument, in dem mindestens ein wärmeerzeugendes Bauteil angeordnet ist und das ein Kopfstück umfasst, das eine Wärmeaustauschfläche aufweist. Innerhalb des medizinischen Instruments kann ein Wärmeüberträger zur Übertragung mindestens eines Teils der von dem mindestens einen wärmeerzeugenden Bauteil beim Betrieb des medizinischen Instruments erzeugten Verlustwärme zur Wärmeaustauschfläche angeordnet sein. Das medizinische Instrument ist zum Verbinden mit einem wie oben beschriebenen Kühlansatz zur Wärmeaufnahme von dem Kopfstück und zur Wärmeabführung mittels eines den Kühlansatz durchströmenden Kühlfluids ausgebildet. Ein erfindungsgemäßes medizinisches Instrument weist insbesondere die oben beschriebenen Merkmale des medizinischen Instruments auf.

Weitere Aspekte der Erfindung ergeben sich aus der nachfolgenden Beschreibung bevorzugter Ausführungsbeispiele und der beigefügten Zeichnung. Es zeigen:
Fig. 1 eine schematische Prinzipdarstellung eines ersten Ausführungsbeispiels einer erfindungsgemäßen endoskopischen und/oder medizinischen Vorrichtung im Längsschnitt;
Fig. 2 einen Längsschnitt durch eine erste Variante der Vorrichtung gemäß dem ersten Ausführungsbeispiel in Detaildarstellung;
Fig. 3 einen Querschnitt durch die in Fig. 2 gezeigte Hülse des Kühlansatzes gemäß einer zweiten Variante des ersten Ausführungsbeispiels;
Fig. 4 einen Längsschnitt durch ein Kopfstück und einen Kühlansatz gemäß einer ersten Variante eines zweiten Ausführungsbeispiels der Erfindung;
Fig. 5 eine zweite Variante des zweiten Ausführungsbeispiels der Erfindung.

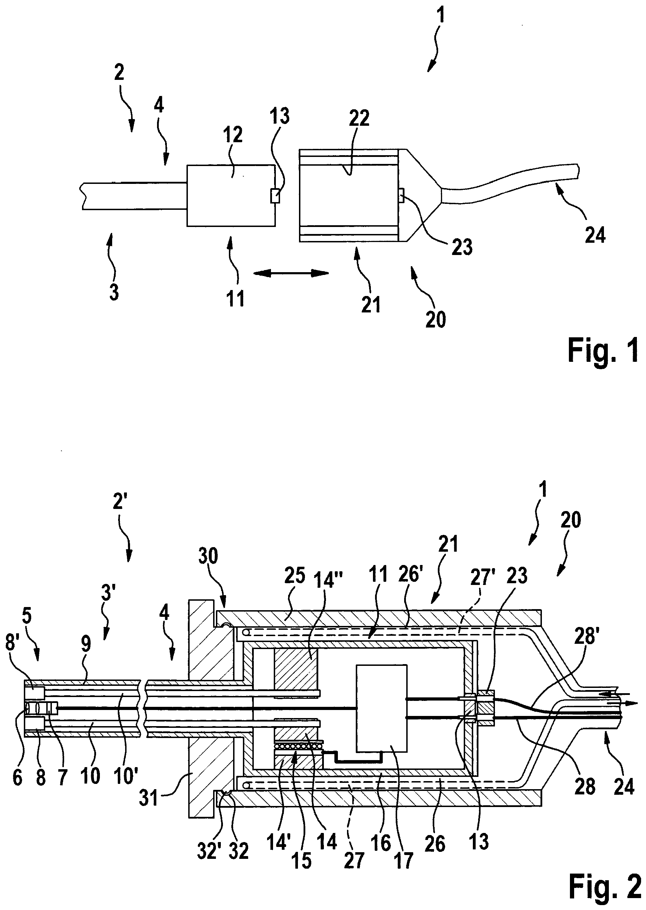

Wie in Fig. 1 schematisch dargestellt, umfasst eine medizinische und/oder endoskopische Vorrichtung 1 gemäß einem ersten Ausführungsbeispiel der Erfindung ein Endoskop bzw. ein medizinisches Instrument 2, das einen lediglich symbolisch dargestellten distalen Bereich 3 und ein an dessen proximalem Ende 4 angeordnetes Kopfstück 11 umfasst. Das Kopfstück 11 ist zylindrisch geformt mit einer einen Zylindermantel bildenden Außenseite 12. Die Vorrichtung 1 umfasst ferner einen als hülsenartigen Stecker ausgebildeten Kühlansatz 20 mit der Hülse 21, wobei deren Innenseite 22 mit der Außenseite 12 des Kopfstücks 11 zum Wärmeaustausch zusammenwirkt. Durch eine in Richtung des Pfeils gerichtete axiale Bewegung kann die Hülse 21 auf das Kopfstück 11 aufgeschoben werden, wodurch die Innenseite 22 der Hülse 21 in Kontakt mit der Außenseite 12 des Kopfstücks 11 oder zumindest in eine nahe benachbarte Anordnung zu dieser gebracht werden kann. In Fig. 1 sind ferner symbolisch die Steckverbinderteile 13, 23 dargestellt, die beim Aufschieben der Hülse 21 auf das Kopfstück 11 zum Herstellen einer elektrischen Verbindung zusammenwirken. Ferner ist in Fig. 1 eine Versorgungsleitung 24 dargestellt, die elektrische Leitungen und Leitungen für das Kühlfluid, das den Kühlansatz 20 durchströmt, enthält.

In der Schnittansicht der Fig. 2 ist eine erste Variante der in Fig. 1 gezeigten Vorrichtung dargestellt. In dieser Variante umfasst die Vorrichtung 1 ein Endoskop 2' und einen Kühlansatz 20. Das Endoskop 2' weist einen langerstreckten Schaft 3' auf, wobei im Bereich des distalen Endes 5 des Schafts 3' elektrische und optische Bauelemente angeordnet sind, insbesondere ein Objektiv 6 zur Erzeugung eines Bilds einer aufgenommenen endoskopischen Szene auf einem Bildsensor 7 und zwei LEDs 8, 8' zur Beleuchtung der aufzunehmenden endoskopischen Szene. Die LEDs 8, 8' erzeugen beim Betrieb Verlustwärme, die, wenn sie nicht abgeführt würde, den Außenschaft 9 zumindest im Bereich des distalen Endes 5 des Schafts 3' unzulässig erwärmen könnte, so dass mit dem Außenschaft 9 in Kontakt kommendes Gewebe beschädigt würde. Zur Abführung der von den LEDs 8, 8' erzeugten Verlustwärme sind Heatpipes 10, 10' vorgesehen, die an ihren distalen Enden mit den LEDs 8, 8' thermisch gekoppelt sind und die innerhalb des Schafts 3' bis über dessen proximales Ende 4 hinaus verlaufen und in das Innere des Kopfstücks 11 hineinreichen. Das Gehäuse 16 des Kopfstücks 11 bildet zusammen mit dem Schaft 3' eine hermetisch dichte Einheit.

Im Kopfstück 11 ist die Heatpipe 10 thermisch über einen Wärmeübertragungsblock 14 aus einem wärmeleitfähigen Material mit einem Peltier-Element 15 gekoppelt, das über einen weiteren Wärmeübertragungsblock 14' thermisch an das Gehäuse 16 des Kopfstücks 11 angekoppelt ist. Das Peltier-Element 15 wird derart betrieben, dass die mit der Heatpipe 10 gekoppelte Seite gekühlt wird und die von der Heatpipe 10 zugeführte Wärme zuzüglich der Verlustwärme des Peltier-Elements 15 an das Gehäuse 16 übertragen wird; hierfür ist die "kalte" Seite des Peltier-Elements 15 mit der Heatpipe 10 und die "warme" Seite des Peltier-Elements 15 mit dem Gehäuse 16 thermisch gekoppelt. Die weitere Heatpipe 10' ist nur über einen Wärmeübertragungsblock 14" mit dem Gehäuse 16 thermisch gekoppelt. Die in der Figur 2 gezeigte Anordnung ist dabei lediglich exemplarisch zu verstehen. Sofern nur eine Heatpipe zur Abführung der im Schaft 3' erzeugten Verlustwärme notwendig ist, kann diese, wie bei der Heatpipe 10 gezeigt, über ein Peltier-Element 15 und ggf. einen oder mehrere Wärmeübertragungsblöcke 14, 14' mit dem Gehäuse 16 thermisch gekoppelt sein oder, wie bei der Heatpipe 10' gezeigt, nur über einen Wärmeleitungsblock 14" an das Gehäuse 16 angekoppelt sein. Sind mehrere Heatpipes zur Abführung der im Schaft 3' erzeugten Verlustwärme vorgesehen, so können diese nach den jeweiligen Anforderungen, insbesondere hinsichtlich der jeweils zu übertragenden Wärmemenge, in gleicher oder unterschiedlicher Weise über ein oder mehrere Peltier-Elemente oder nur über einen oder mehrere Wärmeübertragungsblöcke an das Gehäuse 16 angekoppelt sein.

In der in Fig. 2 gezeigten Anordnung ist der Kühlansatz 20 mit der Hülse 21 über das Kopfstück 11 geschoben. Die Hülse 21 wird durch ein äußeres zylindrisches Rohr 25 gebildet, dessen Innenseite mehrere Kühlkörper 26, 26' trägt, die aus einem weichen Material bestehen, in das Kühlkanäle 27, 27' integriert sind, die von Kühlfluid durchströmt werden können. Als Kühlfluid kann ein flüssiges oder gasförmiges Medium dienen, insbesondere Wasser. Die Kühlkörper 26, 26' liegen mit ihrer Innenseite 22 an der Außenseite 12 des Gehäuses 16 an. Die Außenseite 12 der Gehäuses 16 und die Innenseite 22 der Hülse 21 wirken somit als Wärmeaustauschflächen zur Übertragung der abzuführenden Wärme vom Kopfstück 11 zum Kühlansatz 20 zusammen. Das Material der Kühlkörper 26, 26' weist hierfür eine ausreichende Wärmeleitfähigkeit auf.

An der stirnseitigen Gehäusewand des Kopfstücks 11 ist ein Steckverbinderteil 13 angeordnet, das mit dem Gehäuse 16 hermetisch dicht verbunden ist. In dem Gehäuse 16 des Kopfstücks 11 ist eine Elektronikanordnung 17 untergebracht, die elektrisch mit dem Steckverbinderteil 13 verbunden ist und zur Versorgung des Peltier-Elements 15 sowie der LEDs 8, 8' und des Bildsensors 7 dient. Ferner werden die vom Bildsensor 7 aufgenommenen Bilddaten über die Elektronikanordnung 17 weitergeleitet. Die Steckkontakte der Steckverbinderteile 13 sind in axialer Richtung des Kopfstücks 11 gerichtet, so dass durch Aufschieben des Kühlansatzes 20 mit der Hülse 21 auf das Kopfstück 11 gleichzeitig das Steckverbinderteil 23 des Kühlansatzes 20 in Verbindung mit dem Steckverbinderteil 13 des Kopfstücks 11 gebracht wird. An das Steckverbinderteil 23 des Kühlansatzes 20 sind elektrische Leitungen 28, 28' angeschlossen, die eine Verbindung mit einer nicht dargestellten Versorgungseinrichtung herstellen. Die elektrischen Leitungen 28, 28' und die Fluidleitungen 29, 29' zur Zu- und Abführung des Kühlfluids sind in einer Versorgungsleitung 24 geführt. Die übrigen Bereiche der Kühlfluidversorgung, beispielsweise eine Pumpe oder ein Sterilwasserbeutel, sind in Fig. 2 ebenfalls nicht gezeigt.

In Fig. 2 ist ferner symbolisch eine Verriegelungseinrichtung 30 dargestellt. Die Verriegelungseinrichtung 30 kann beispielsweise am äußeren Rohr 25 und an einer mit dem Endoskop 2' verbundenen Halterung 31 angeordnete Riegelelemente 32, 32' umfassen, die durch Relativdrehung des Endoskops 2' und des Kühlansatzes 20 formschlüssig miteinander in Eingriff gebracht und voneinander gelöst werden können. Hierdurch kann der Kühlansatz 20 sicher mit dem Endoskop 2' verbunden und wieder von diesem getrennt werden.

Anstelle des Endoskops 2' kann die in Fig. 2 dargestellte Variante eines Ausführungsbeispiels einer erfindungsgemäßen Vorrichtung 1 ein medizinisches Instrument umfassen (nicht dargestellt).

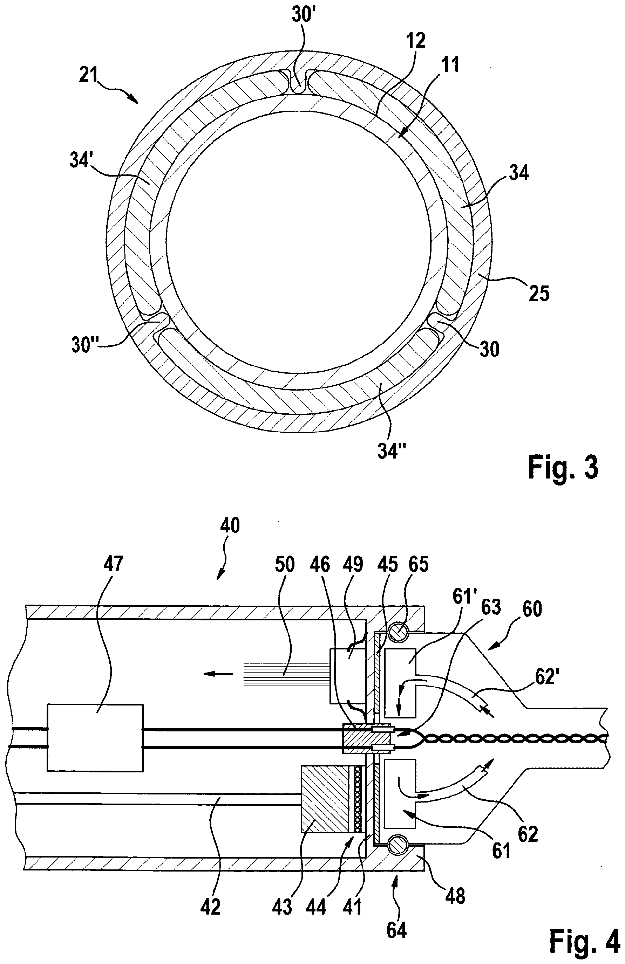

Wie in Fig. 3 in einem schematischen Querschnitt gezeigt, kann bei einer zweiten Variante des in Fig. 2 dargestellten Ausführungsbeispiels die Hülse 21 auf ihrer Innenseite eine Mehrzahl an leistenförmigen Einführnasen 33, 33', 33" aufweisen, die möglichst passgenau ein Einführen des Kopfstücks 11 in die Hülse 21 ermöglichen, wobei zwischen den Einführnasen 33, 33', 33" auf der Innenseite des Rohrs 25 mit Kühlfluid befüllbare, volumenveränderliche Kühlkörper 34, 34', 34" aus einem elastischen Material angeordnet sind. Während die Kühlkörper 34, 34', 34" in einem nicht befüllten Zustand ein leichtes Einschieben des in Fig. 3 symbolisch angedeuteten Kopfstücks 11 ermöglichen, liegen die Kühlkörper 34, 34', 34" im mit Kühlfluid befüllten Zustand eng an der Außenseite 12 des Kopfstücks 11 an. Die Kühlkörper 34, 34', 34" können auch als Ballons ausgeführt sein. Das Rohr 25 kann als Griffschale eines Handgriffs ausgebildet sein oder eine solche tragen. Im Übrigen kann die zweite Variante wie die in Fig. 2 dargestellte erste Variante des ersten Ausführungsbeispiels ausgebildet sein, kann aber auch beispielsweise ein medizinisches Instrument umfassen.

Bei der in Fig. 4 in einem schematischen Längsschnitt dargestellten ersten Variante eines zweiten Ausführungsbeispiels der Erfindung wirkt die Stirnwand 41 des Kopfstücks 40 als Wärmeaustauschfläche. Die Verlustwärme, die von einem im distalen Endbereich eines Endoskops, das wie zu Fig. 2 beschrieben ausgebildet ist, oder im distalen Bereich eines medizinischen Instruments angeordneten wärmeerzeugenden Bauteil erzeugt wird, wird über eine Heatpipe 42 zu einem mit der Heatpipe 42 über einen Wärmeübertragungsblock 43 gekoppelten Peltier-Element 44 geleitet, das an die Stirnwand 41 thermisch angekoppelt ist; da die Stirnwand 41 flach ausgebildet ist, ist eine direkte Ankopplung des Peltier-Elementes 44 an die Stirnwand 41 möglich. An der Stirnwand 41 ist zumindest bereichsweise eine Schicht 45 aus einem wärmeleitfähigen elastischen Material aufgebracht. Der Kühlansatz 60 weist eine Wärmetauscherkammer 61 auf, die von Kühlfluid durchströmbar ist und die dem Bereich der Stirnwand 41, an den das Peltier-Element 44 angekoppelt ist, gegenüberliegend angeordnet ist. Die Wärmetauscherkammer 61 kann mit einer oder mehreren weiteren Wärmetauscherkammern 61', die anderen Bereichen der Stirnwand 41 gegenüberliegend angeordnet sind, über eine aus Gründen der Übersichtlichkeit nicht dargestellte Leitung verbunden sein und durch Fluidleitungen 62, 62' von einer nicht dargestellten Versorgungseinrichtung versorgt werden, wobei eine mögliche Flussrichtung durch die Pfeile angedeutet ist. Ein Wärmeaustausch von der Stirnwand 41 zum Kühlfluid erfolgt über die Schicht 45 zu den Wärmetauscherkammern 61, 61', aus denen die Wärme durch das Kühlfluid abgeführt wird. Die Stirnwand 41 trägt ferner ein Steckverbinderteil 46, das mit einem Steckverbinderteil 63 des Kühlansatzes 60 wie oben zu Fig. 1 und 2 beschrieben zur Herstellung einer elektrischen Verbindung zwischen einer Elektronikanordnung 46 und der Versorgungseinrichtung zusammenwirkt. In Fig. 4 ist symbolisch eine Verriegelungseinrichtung 64 dargestellt, die Riegelelemente 65 umfasst, die in Ausnehmungen auf der Innenseite einer axialen Verlängerung 48 des zylindrischen Gehäuses des Kopfstücks 40 und auf der Außenseite des Kühlansatzes 60 lösbar eingreifen. Die Verriegelungseinrichtung 64 ermöglicht eine Befestigung des Kühlansatzes 60 am Kopfstück 40 und die Ausübung einer für den Wärmeaustausch ausreichenden Andruckkraft an die Stirnwand 41. Toleranzen der Verriegelung werden durch die Schicht 45 ausgeglichen.

Anstelle oder zusätzlich zu einer im distalen Endbereich des Endoskops bzw. des medizinischen Instruments angeordneten Wärmequelle kann auch eine Wärmequelle, beispielsweise eine LED 49, im Kopfstück 40 angeordnet sein. Bei einem Endoskop mit einer im Kopfstück 40 angeordneten LED 49 ist ein Lichtleiter, etwa eine Glasfaseroptik 50, zur Weiterleitung des von der LED 49 erzeugten Lichts zum distalen Ende des Schafts und zur Beleuchtung der endoskopischen Szene vorgesehen (durch den Pfeil angedeutet). Die LED 49 kann direkt an der Stirnwand 41 angeordnet und mit dieser thermisch gekoppelt sein. Bei einem medizinischen Instrument kann beispielsweise anstelle der LED 49 ein Motor oder ein anderes wärmeerzeugendes Element angeordnet sein, das ebenfalls mit der Stirnwand 41 thermisch gekoppelt ist. Anstelle des Lichtleiters kann in diesem Fall etwa eine Welle zur Weiterleitung der von dem Motor erzeugten Bewegung in den distalen Bereich des Instruments vorgesehen sein. Durch die weitere Wärmetauscherkammer 61' wird die von dem wärmeerzeugenden Bauteil, insbesondere der LED 49, erzeugte Verlustwärme abgeführt. Es versteht sich, dass die Darstellung der Fig. 4 nur exemplarisch eine Anordnung mit einer Heatpipe 42 zur Weiterleitung der im Bereich des distalen Endes des Endoskops erzeugten Verlustwärme zum Kopfstück 40 und mit einer im Kopfstück 40 angeordneten Licht- bzw. Wärmequelle (LED 49) gezeigt ist. So ist es beispielsweise auch denkbar, dass ein Endoskop bzw. ein medizinisches Instrument ausschließlich über im Bereich des distalen Endes angeordnete Licht- bzw. Wärmequellen verfügt oder ausschließlich über im Kopfstück 40 angeordnete Licht- bzw. Wärmequellen mit jeweils entsprechend ausgebildeter Wärmeabführung. Das Gleiche gilt auch bei dem in dem Figuren 1 bis 3 dargestellten ersten Ausführungsbeispiel der Erfindung.

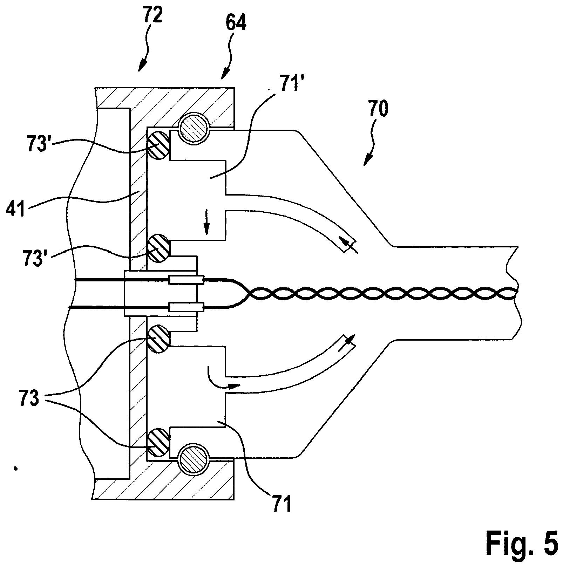

In Fig. 5 ist eine zweite Variante des zweiten Ausführungsbeispiels der Erfindung gezeigt. Dabei sind anstelle der in der ersten Variante gemäß Fig. 4 vorgesehenen Wärmetauscherkammern 61, 61' zur Stirnwand 41 hin offene Fluidkammern 71, 71' angeordnet. Wenn der Kühlansatz 70 an das Kopfstück 72 angesetzt wird, werden die Fluidkammern 71, 71' durch die Stirnwand 41 selbst sowie durch Dichtungen, die die Fluidkammern 71, 71' seitlich abschließen, fluiddicht begrenzt. Die Dichtungen können beispielsweise als Dichtringe 73, 73' ausgebildet sein. Hierbei kommt das Kühlfluid in direkten Kontakt mit der metallischen Stirnwand 41; auf eine wärmeleitfähige Schicht aus elastischem Material auf der Stirnwand 41 kann dabei verzichtet werden. Im Übrigen sind der Kühlansatz 70 und das Kopfstück 72 wie der zu Fig. 4 beschriebene Kühlansatz 60 und das Kopfstück 40 ausgebildet, wobei die Verriegelungseinrichtung 64 die Ausübung einer für die Dichtwirkung der Dichtungen ausreichenden Andruckkraft an die Stirnwand 41 ermöglicht.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Vorrichtung
- 2: Endoskop bzw. medizinisches Instrument
- 2': Endoskop
- 3: Distaler Bereich
- 3': Schaft
- 4: Proximales Ende
- 5: Distales Ende
- 6: Objektiv
- 7: Bildsensor
- 8, 8': LED
- 9: Außenschaft
- 10,: 10'Heatpipe
- 11: Kopfstück
- 12: Außenseite
- 13: Steckverbinderteil
- 14, 14', 14": Wärmeübertragungsblock
- 15: Peltier-Element
- 16: Gehäuse
- 20: Kühlansatz
- 21: Hülse
- 22: Innenseite
- 23: Steckverbinderteil
- 24: Versorgungsleitung
- 25: Rohr
- 26, 26': Kühlkörper
- 27, 27': Kühlkanal
- 28, 28': Elektrische Leitung
- 29, 29': Fluidleitung
- 30: Verriegelungseinrichtung
- 31: Halterung
- 32, 32': Riegelelement
- 33, 33', 33": Einführnase
- 34, 34', 34": Kühlkörper
- 40: Kopfstück
- 41: Stirnwand
- 42: Heatpipe
- 43: Wärmeübertragungsblock
- 44: Peltier-Element
- 45: Schicht
- 46: Steckverbinderteil
- 47: Elektronikanordnung
- 48: Verlängerung
- 49: LED
- 50: Glasfaseroptik
- 60: Kühlansatz
- 61, 61': Wärmetauscherkammer
- 62, 62': Fluidleitung
- 63: Steckverbinderteil
- 64: Verriegelungseinrichtung
- 65: Riegelelement
- 70: Kühlansatz
- 71, 71': Fluidkammer
- 72: Kopfstück
- 73, 73': Dichtring

## Patentansprüche

1. Kühleinrichtung für ein Endoskop (2') oder ein medizinisches Instrument, wobei das Endoskop (2') und/oder das medizinische Instrument ein Kopfstück (11, 40, 72) und einen lang erstreckten Schaft (3') aufweist, wobei das Kopfstück (11, 40, 72) an einem proximalen Endbereich des Schafts (3') angeordnet ist, wobei in dem Endoskop (2') und/oder in dem medizinischen Instrument mindestens ein wärmeerzeugendes Bauteil angeordnet ist und wobei das Kopfstück (11, 40, 72) eine einen Teil der Oberfläche eines Gehäuses des Kopfstücks bildende Wärmeaustauschfläche zur Abgabe der von dem mindestens einen wärmeerzeugenden Bauteil erzeugten Wärme nach außerhalb des Endoskops (2') und/oder des medizinischen Instruments aufweist, wobei die Kühleinrichtung als mit dem Endoskop (2') und/oder mit dem medizinischen Instrument lösbar verbindbarer Kühlansatz (20, 60, 70) ausgebildet ist, der zur Wärmeaufnahme von der Wärmeaustauschfläche des Kopfstücks (11, 40, 72) und zur Wärmeabführung durch ein den Kühlansatz (20, 60, 70) durchströmendes Kühlfluid ausgebildet ist, wobei der Kühlansatz (20, 60, 70) zur Durchströmung mit einem in einem geschlossenen Kühlkreislauf geführten Kühlfluid ausgebildet ist und wobei der Kühlansatz (20, 60, 70) als Handgriff ausgebildet ist, **dadurch gekennzeichnet, dass** die Wärmeaustauschfläche des Kopfstückes (11, 40, 72) einen axial gerichteten Zylinder bildet und der Kühlansatz (20, 60, 70) als auf den Zylinder aufschiebbare Hülse (21) ausgebildet ist.

2. Endoskopische und/oder medizinische Vorrichtung mit einem Endoskop (2') und/oder mit einem medizinischen Instrument, wobei das Endoskop (2') und/oder das medizinische Instrument ein Kopfstück (11, 40, 72) und einen lang erstreckten Schaft (3') aufweist, wobei das Kopfstück (11, 40, 72) an einem proximalen Endbereich des Schafts (3') angeordnet ist, wobei in dem Endoskop (2') und/oder in dem medizinischen Instrument mindestens ein wärmeerzeugendes Bauteil angeordnet ist und wobei das Kopfstück (11, 40, 72) eine einen Teil der Oberfläche eines Gehäuses des Kopfstücks bildende Wärmeaustauschfläche zur Abgabe der von dem mindestens einen wärmeerzeugenden Bauteil erzeugten Wärme nach außerhalb des Endoskops (2') und/oder des medizinischen Instruments aufweist, **dadurch gekennzeichnet, dass** die Vorrichtung (1) eine Kühleinrichtung nach Anspruch 1 umfasst, wobei die Wärmeaustauschfläche des Kopfstückes (11, 40, 72) einen axial gerichteten Zylinder ausbildet.

3. Vorrichtung nach Anspruch 2, **dadurch gekennzeichnet, dass** in einem distalen Endbereich des Schafts (3') mindestens ein wärmeerzeugendes Bauteil und innerhalb des Schafts (3') ein Wärmeüberträger zur Übertragung der von dem mindestens einen wärmeerzeugenden Bauteil erzeugten Wärme zu einer Wärmeaustauschfläche des Kopfstücks (11, 40, 72) angeordnet sind.

4. Vorrichtung nach Anspruch 2 oder 3, **dadurch gekennzeichnet, dass** das Endoskop (2') und/oder das medizinische Instrument hermetisch dicht ausgebildet ist.

5. Vorrichtung nach einem der Ansprüche 2 bis 4, **dadurch gekennzeichnet, dass** der Kühlansatz (20, 60, 70) als Einwegteil ausgebildet ist.

6. Vorrichtung nach einem der vorhergehenden Ansprüche, **dadurch gekennzeichnet, dass** der Kühlansatz (20, 60, 70) zur Durchströmung mit Kühlfluid unter Schwerkraftwirkung ausgebildet ist.

7. Vorrichtung nach einem der vorhergehenden Ansprüche, **dadurch gekennzeichnet, dass** der Kühlansatz (20, 60, 70) eine Wärmeaustauschfläche aufweist, die mit der Wärmeaustauschfläche des Kopfstücks (11, 40, 72) zumindest teilweise in thermischem Kontakt bringbar ist und die mit einem von dem Kühlfluid durchströmbaren Wärmetauscher thermisch gekoppelt ist.

8. Vorrichtung nach Anspruch 7, **dadurch gekennzeichnet, dass** die Wärmeaustauschfläche des Kopfstücks (11, 40, 72) als radiale äußere Oberfläche ausgebildet ist.

9. Vorrichtung nach Anspruch 7 oder 8, **dadurch gekennzeichnet, dass** die Wärmeaustauschfläche des Kopfstücks (11, 40, 72) mindestens einen Teil einer proximalen Stirnseite des Kopfstücks (2) bildet.

10. Vorrichtung nach einem der Ansprüche 7 bis 9, **dadurch gekennzeichnet, dass** in einem nach dem Verbinden des Kühlansatzes (20, 60, 70) mit dem Endoskop (2') und/oder mit dem medizinischen Instrument verbleibenden Spalt zwischen der Wärmeaustauschfläche des Kopfstücks (11, 40, 72) und der Wärmeaustauschfläche des Kühlansatzes (20, 60, 70) ein wärmeleitfähiges Material angeordnet ist, das bei einer Erwärmung im Betrieb der Vorrichtung (1) eine zum Ausfüllen des Spalts ausreichende Volumenvergrößerung erfährt.

11. Vorrichtung nach einem der Ansprüche 2 bis 10, **dadurch gekennzeichnet, dass** in einem nach dem Verbinden des Kühlansatzes (20, 60, 70) mit dem Endoskop (2') und/oder mit dem medizinischen Instrument verbleibenden Spalt zwischen der Wärmeaustauschfläche des Kopfstücks (11, 40, 72) und einer Oberfläche des Kühlansatzes (20, 60, 70) mindestens ein mit Kühlfluid befüllbares volumenveränderliches Element angeordnet ist.

12. Vorrichtung nach einem der Ansprüche 2 bis 11, **dadurch gekennzeichnet, dass** der Kühlansatz (20, 60, 70) zu einer derartigen fluiddichten Verbindung mit dem Kopfstück (11, 40, 72) ausgebildet ist, dass zwischen der Wärmeaustauschfläche des Kopfstücks (11, 40, 72) und einer Oberfläche des Kühlansatzes (20, 60, 70) ein von dem Kühlfluid durchströmbarer Raum geschaffen wird.

13. Vorrichtung nach einem der vorhergehenden Ansprüche, **dadurch gekennzeichnet, dass** das Kopfstück (11, 40, 72) und der Kühlansatz (20, 60, 70) miteinander zusammenwirkende Steckverbinderteile (13, 23, 46, 63) zum Herstellen einer elektrischen Verbindung zwischen dem Endoskop (2') und/oder dem medizinischen Instrument und dem Kühlansatz (20, 60, 70) aufweisen.

## Claims

1. Cooling means for an endoscope (2') or a medical instrument, wherein the endoscope (2') and/or the medical instrument comprises a head piece (11, 40, 72) and an elongated shaft (3'), wherein the head piece (11, 40, 72) is arranged on a proximal end area of the shaft (3'), wherein arranged in the endoscope (2') and/or in the medical instrument is at least one heat-generating component and wherein the head piece (11, 40, 72) comprises a heat exchange surface forming a part of the surface of a housing of the head piece for dissipating the heat generated by the at least one heat-generating component to outside of the endoscope (2') and/or of the medical instrument, wherein the cooling means is formed as a cooling attachment (20, 60, 70), which is connectable detachably to the endoscope (2') and/or to the medical instrument and is designed to absorb heat from the heat exchange surface of the head piece (11, 40, 72) and to dissipate heat through a cooling fluid flowing through the cooling attachment (20, 60, 70), wherein the cooling attachment (20, 60, 70) is designed for through-flow by a cooling fluid carried in a closed cooling circuit and wherein the cooling attachment (20, 60, 70) is designed as a handle, **characterised in that** the heat exchange surface of the head piece (11, 40, 72) forms an axially directed cylinder and the cooling attachment (20, 60, 70) is designed as a sleeve (21) that can be pushed onto the cylinder.

2. Endoscopic and/or medical device with an endoscope (2') or with a medical instrument, wherein the endoscope (2') and/or the medical instrument has a head piece (11, 40, 72) and an elongated shaft (3'), wherein the head piece (11, 40, 72) is arranged on a proximal end area of the shaft (3'), wherein arranged in the endoscope (2') and/or in the medical instrument is at least one heat-generating component and wherein the head piece (11, 40, 72) has a heat exchange surface forming a part of the surface of a housing of the head piece for dissipating the heat generated by the at least one heat-generating component to outside of the endoscope (2') and/or of the medical instrument, **characterised in that** the device (1) comprises a cooling means according to claim 1, wherein the heat exchange surface of the head piece (11, 40, 72) forms an axially directed cylinder.

3. Device according to claim 2, **characterised in that** there are arranged in a distal end area of the shaft (3') at least one heat-generating component and inside the shaft (3') a heat exchanger for transmitting the heat generated by the at least one heat-generating component to a heat exchange surface of the head piece (11, 40, 72).

4. Device according to claim 2 or 3, **characterised in that** the endoscope (2') and/or the medical instrument is designed hermetically sealed.

5. Device according to any one of claims 2 to 4, **characterised in that** the cooling attachment (20, 60, 70) is designed as a disposable part.

6. Device according to any one of the preceding claims, **characterised in that** the cooling attachment (20, 60, 70) is designed for through-flow by cooling fluid under the effect of gravity.

7. Device according to any one of the preceding claims, **characterised in that** the cooling attachment (20, 60, 70) comprises a heat exchange surface, which can be brought at least partly into thermal contact with the heat exchange surface of the head piece (11, 40, 72) and which is thermally coupled to a heat exchanger through which the cooling fluid can flow.

8. Device according to claim 7, **characterised in that** the heat exchange surface of the head piece (11, 40, 72) is designed as a radial outer surface.

9. Device according to claim 7 or 8, **characterised in that** the heat exchange surface of the head piece (11, 40, 72) forms at least a part of a proximal end face of the head piece (2).

10. Device according to any one of claims 7 to 9, **characterised in that** arranged in a gap remaining between the heat exchange surface of the head piece (11, 40, 72) and the heat exchange surface of the cooling attachment (20, 60, 70) after connection of the cooling attachment (20, 60, 70) to the endoscope (2') and/or to the medical instrument is a thermally conductive material, which in the event of heating in operation of the device (1) experiences a volume enlargement sufficient to fill the gap.

11. Device according to any one of claims 2 to 10, **characterised in that** arranged in a gap remaining between the heat exchange surface of the head piece (11, 40, 72) and a surface of the cooling attachment (20, 60, 70) after connection of the cooling attachment (20, 60, 70) to the endoscope (2') and/or to the medical instrument is at least one volume-variable element that can be filled with cooling fluid.

12. Device according to any one of claims 2 to 11, **characterised in that** the cooling attachment (20, 60, 70) is formed for a fluid-tight connection to the head piece (11, 40, 72) such that a space through which the cooling fluid can flow is created between the heat exchange surface of the head piece (11, 40, 72) and a surface of the cooling attachment (20, 60, 70).

13. Device according to any one of the preceding claims, **characterised in that** the head piece (11, 40, 72) and the cooling attachment (20, 60, 70) have plug connector parts (13, 23, 46, 63) interacting with one another to create an electrical connection between the endoscope (2') and/or the medical instrument and the cooling attachment (20, 60, 70).

## Revendications

1. Moyen de refroidissement pour un endoscope (2') ou un instrument médical, dans lequel l'endoscope (2') et/ou l'instrument médical présentent une partie tête (11, 40, 72) et un manche (3') étiré en longueur, dans lequel la partie tête (11, 40, 72) est disposée sur une zone d'extrémité proximale du manche (3'), dans lequel au moins un composant de production de chaleur est disposé dans l'endoscope (2') et/ou dans l'instrument médical et dans lequel la partie tête (11, 40, 72) présente une face d'échange de chaleur formant une partie de la surface d'un boîtier de la partie tête pour dissiper la chaleur produite par l'au moins un composant de production de chaleur vers l'extérieur de l'endoscope (2') et/ou de l'instrument médical, dans lequel le moyen de refroidissement est réalisé en tant qu'embout de refroidissement (20, 60, 70) pouvant être raccordé de manière amovible à l'endoscope (2') et/ou à l'instrument médical, qui est réalisé pour l'absorption de chaleur de la face d'échange de chaleur de la partie tête (11, 40, 72) et pour la dissipation de chaleur par un fluide de refroidissement traversant l'embout de refroidissement (20, 60, 70), dans lequel l'embout de refroidissement (20, 60, 70) est réalisé pour être traversé par un fluide de refroidissement guidé dans un circuit de refroidissement fermé et dans lequel l'embout de refroidissement (20, 60, 70) est réalisé en tant que poignée, **caractérisé en ce que** la face d'échange de chaleur de la partie tête (11, 40, 72) forme un cylindre dirigé de manière axiale et l'embout de refroidissement (20, 60, 70) est réalisé en tant que manchon (21) pouvant être enfilé sur le cylindre.

2. Dispositif endoscopique et/ou médical avec un endoscope (2') et/ou avec un instrument médical, dans lequel l'endoscope (2') et/ou l'instrument médical présentent une partie tête (11, 40, 72) et un manche (3') étiré en longueur, dans lequel la partie tête (11, 40, 72) est disposée sur une zone d'extrémité proximale du manche (3'), dans lequel au moins un composant de production de chaleur est disposé dans l'endoscope (2') et/ou dans l'instrument médical et dans lequel la partie tête (11, 40, 72) présente une face d'échange de chaleur formant une partie de la surface d'un boîtier de la partie tête, pour dissiper la chaleur produite par l'au moins un composant de production de chaleur vers l'extérieur de l'endoscope (2') et/ou de l'instrument médical, **caractérisé en ce que** le dispositif (1) comprend un moyen de refroidissement selon la revendication 1, dans lequel la face d'échange de chaleur de la partie tête (11, 40, 72) réalise un cylindre dirigé de manière axiale.

3. Dispositif selon la revendication 2, **caractérisé en ce qu'**au moins un composant de production de chaleur est disposé dans une zone d'extrémité distale du manche (3') et un échangeur de chaleur pour transférer la chaleur produite par l'au moins un composant de production de chaleur à une face d'échange de chaleur de la partie tête (11, 40, 72) est disposé à l'intérieur du manche (3').

4. Dispositif selon la revendication 2 ou 3, **caractérisé en ce que** l'endoscope (2') et/ou l'instrument médical sont réalisés de manière hermétiquement étanche.

5. Dispositif selon l'une quelconque des revendications 2 à 4, **caractérisé en ce que** l'embout de refroidissement (20, 60, 70) est réalisé en tant que partie à usage unique.

6. Dispositif selon l'une quelconque des revendications précédentes, **caractérisé en ce que** l'embout de refroidissement (20, 60, 70) est réalisé pour être traversé avec du fluide de refroidissement sous l'effet de la gravité.

7. Dispositif selon l'une quelconque des revendications précédentes, **caractérisé en ce que** l'embout de refroidissement (20, 60, 70) présente une face d'échange de chaleur, qui peut être amenée au moins en partie en contact thermique avec la face d'échange de chaleur de la partie tête (11, 40, 72) et qui est couplée de manière thermique à un échangeur de chaleur pouvant être traversé par le fluide de refroidissement.

8. Dispositif selon la revendication 7, **caractérisé en ce que** la face d'échange de chaleur de la partie tête (11, 40, 72) est réalisée en tant que surface extérieure radiale.

9. Dispositif selon la revendication 7 ou 8, **caractérisé en ce que** la face d'échange de chaleur de la partie tête (11, 40, 72) forme au moins une partie d'un côté frontal proximal de la partie tête (2).

10. Dispositif selon l'une quelconque des revendications 7 à 9, **caractérisé en ce qu'**est disposé dans une fente, restant après le raccordement de l'embout de refroidissement (20, 60, 70) à l'endoscope (2') et/ou à l'instrument médical, entre la face d'échange de chaleur de la partie tête (11, 40, 72) et la face d'échange de chaleur de l'embout de refroidissement (20, 60, 70), un matériau thermoconducteur, qui subit un agrandissement de volume suffisant pour remplir la fente lors d'un réchauffement lors du fonctionnement du dispositif (1).

11. Dispositif selon l'une quelconque des revendications 2 à 10, **caractérisé en ce qu'**au moins un élément à volume variable pouvant être rempli de fluide de refroidissement est disposé dans une fente, restant après le raccordement de l'embout de refroidissement (20, 60, 70) à l'endoscope (2') et/ou à l'instrument médical, entre la face d'échange de chaleur de la partie tête (11, 40, 72) et une surface d'embout de refroidissement (20, 60, 70).

12. Dispositif selon l'une quelconque des revendications 2 à 11, **caractérisé en ce que** l'embout de refroidissement (20, 60, 70) est configuré pour un raccordement étanche aux fluides à la partie tête (11, 40, 72) de telle manière qu'un espace pouvant être traversé par le fluide de refroidissement est créé entre la face d'échange de chaleur de la partie tête (11, 40, 72) et une surface de l'embout de refroidissement (20, 60, 70).

13. Dispositif selon l'une quelconque des revendications précédentes, **caractérisé en ce que** la partie tête (11, 40, 72) et l'embout de refroidissement (20, 60, 70) présentent des parties de raccordement par enfichage (13, 23, 46, 63) coopérant entre elles pour établir une connexion électrique entre l'endoscope (2') et/ou l'instrument médical et l'embout de refroidissement (20, 60, 70).
